# FASCICULE DE BREVET EUROPEEN

(11) **EP 4 425 504 B1**
(45) Date de publication et mention de la délivrance du brevet: **12.08.2026**
(21) Numéro de dépôt: 24156679.3
(22) Date de dépôt: 08.02.2024
(51) Int. Cl.: G16H 20/40, A61M 16/00, A61M 16/10, G16H 40/67

(54) **BOÎTIER DE SURVEILLANCE D'UNE INSTALLATION D' ACHEMINEMENT DE FLUIDES MÉDICAUX EMBARQUANT DES MOYENS DE COMMUNICATION AMOVIBLES**
ÜBERWACHUNGSGEHÄUSE EINER MEDIZINISCHEN FLÜSSIGKEITSLEITANLAGE MIT ABNEHMBAREN KOMMUNIKATIONSMITTELN
MONITORING BOX FOR A MEDICAL FLUID-CONVEYING INSTALLATION INCLUDING REMOVABLE COMMUNICATION MEANS

(30) Priorité: 01.03.2023 FR 2301901
(43) Date de publication de la demande: 04.09.2024
(73) Titulaire: Air Liquide Medical Systems, 92182 Antony Cedex (FR)
(72) Inventeur: BUSSON, Thibaut, 92182 Antony (FR); BEAUCHER, Jérôme, 92182 Antony (FR)
(74) Mandataire: Air Liquide

(56) Documents cités:
- AU-A1- 2018 202 754
- CN-A- 105 548 257
- CN-A- 106 233 350
- CN-U- 210 322 155
- US-A1- 2018 335 038

## Description

La présente invention concerne une installation d'acheminement de fluide dans un établissement ou bâtiment hospitalier, tel un hôpital ou analogue, comprenant un boitier ou appareil modulable de surveillance d'un ou plusieurs fluides médicaux, tels un ou des gaz ou le vide médical (i.e. dépression) véhiculés par une ou des conduites de fluide agencées dans le bâtiment hospitalier, lequel boitier peut accueillir différents modules de télécommunication amovibles, c'est-à-dire détachables et opérant selon les protocoles de communication différents, et est destiné à coopérer avec un équipement de surveillance distance, tel une GTC, typiquement lui transmettre des données ou informations via le ou lesdits modules de télécommunication amovibles.

Les boîtiers de surveillance des fluides médicaux sont des éléments de sécurité obligatoires qui permettent de surveiller, i.e. monitorer, les réseaux de distribution des fluides médicaux, typiquement des lignes ou canalisations d'acheminement des gaz médicaux, tel l'air, l'oxygène ou le protoxyde d'azote (N₂O), ou du vide (i.e. dépression), agencés dans les établissements hospitaliers, tels les hôpitaux, cliniques ou analogues.

Un boîtier de surveillance est habituellement installé sur un mur, une paroi ou analogue, de l'établissement hospitalier considéré, par exemple dans la ou les zones de production des fluides médicaux et/ou à l'entrée d'un ou plusieurs services hospitaliers.

Un ou des capteurs de pression sont reliés aux différentes lignes du réseau de fluides médicaux à surveiller et par ailleurs connectés électriquement au boîtier de surveillance, en particulier aux moyens de pilotage du boitier, telle une carte électronique principale comprenant un ou des microprocesseurs, pour leur fournir les mesures de pression opérées afin qu'elles y soient traitées informatiquement.

Plus précisément, chaque capteur de pression est relié à des moyens de raccordement électrique du boitier, tel un bornier, c'est-à-dire un ensemble de connecteurs électriques, eux-mêmes connectés aux moyens de pilotage du boitier, via des liaisons électriques, aussi appelées voies de surveillance du boîtier de surveillance.

Chaque voie de surveillance est paramétrée de manière spécifique afin d'assurer une surveillance correcte du réseau de fluides et déclencher une alarme lorsqu'une anomalie est détectée, typiquement un franchissement de valeur-seuil de pression d'alerte par exemple.

Lorsqu'une alarme se déclenche, l'information ou l'alerte correspondante peut être simplement affichée sur le boitier ou alors transmise à un équipement de télésurveillance distant, comme une Gestion Technique Centralisée (GTC) servant à monitorer à distance plusieurs appareils au sein d'un établissement hospitalier, tels que boîtiers de surveillance des fluides médicaux, boitiers d'alarme incendie, système de ventilation, système de chauffage et/ou climatisation...

Pour ce faire, les boîtiers de surveillance des fluides médicaux sont équipés de moyens de télécommunication, sachant que la communication proprement-dite peut se faire selon des protocoles de communication différents.

On connait par exemple les documents CN106233350, CN210322155, CN105548257 et AU2018202754.

Dans un autre domaine, US20180335038 décrit une pompe industrielle motorisée comprenant un module de contrôle du moteur de la pompe et des modules enfichables dans le boitier de pompe permettant, lors des maintenances, de communiquer avec le module de contrôle du moteur, d'afficher des informations, contrôler la pompe et/ou opérer des diagnostics. Ces modules enfichables ne servent pas à surveiller la pompe à distance.

Un problème est que, pour pouvoir répondre aux différentes configurations des installations d'acheminement de fluide hospitalières, il convient d'intégrer dans le boitier de l'installation d'acheminement de fluide, les différents protocoles de communication possibles ou alors un système convertisseur pour transformer le protocole de communication du boitier pour le rendre compatible avec les différents équipements de télésurveillance distants (e.g. GTC) existants. Dans les deux cas, ces éléments supplémentaires engendrent inévitablement des surcoûts pouvant être importants.

Un autre problème est que, lorsqu'un hôpital souhaite passer d'un boitier non-communiquant à un boitier avec moyens de télécommunication ou changer de protocole de communication, il doit changer de boitier, ce qui, là encore, engendre des coûts de remplacement, d'installation et autres. De plus, cette opération rend le système indisponible et donc aussi l'installation d'acheminement de fluide, pendant toute la durée du remplacement.

La présente invention s'inscrit dans ce contexte et propose une installation d'acheminement de fluide agencée dans un établissement hospitalier équipée d'un boitier de surveillance de fluide modulable pouvant fonctionner avec ou sans moyens de télécommunication internes, et selon différents protocoles de communication de sorte de résoudre tout ou partie des problèmes susmentionnés.

L'invention concerne alors une installation d'acheminement de fluide dans un établissement hospitalier comprenant un boitier de surveillance de fluide relié électriquement à un ou des capteurs de pression, agencés sur une ou des lignes d'acheminement de fluide, en particulier les lignes d'un réseau de fluides hospitalier,
ledit boitier de surveillance de fluide comprenant :
- des moyens de pilotage comprenant une carte électronique principale comprenant au moins un processeur,
- des moyens de raccordement électrique configurés pour y raccorder électriquement au moins un capteur de pression configuré pour opérer une ou des mesures de pression de fluide, et
- des liaisons électriques pour raccorder électriquement les moyens de raccordement électrique aux moyens de pilotage de manière à fournir aux moyens de pilotage, la ou les mesures de pression provenant du ou des capteur de pression.

De plus, selon l'invention, la carte électronique principale du boitier comprend un seul emplacement à module configuré pour permettre d'y raccorder différents modules de télécommunication amovibles, chaque module amovible comprenant au moins une carte électronique secondaire portant des moyens de télécommunication, ledit module de télécommunication étant configuré pour coopérer, via la carte électronique secondaire, avec les moyens de pilotage comprenant la carte électronique principale pour transmettre, par exemple fournir, émettre ou analogue, une ou des informations (i.e. données, mesures, signal d'alarme...) vers au moins un équipement de surveillance distant, c'est-à-dire un emplacement unique et commun servant au raccordement de modules opérant selon les protocoles de communication différents.

Selon le mode de réalisation considéré, le boitier de l'installation de l'invention peut comprendre l'une ou plusieurs des caractéristiques suivantes :
- les moyens de pilotage, en particulier la carte électronique principale, comprennent un ou plusieurs (micro)processeurs.
- les moyens de raccordement électrique comprennent un ou des connecteurs électriques, en particulier un ensemble de connecteurs ou bornier comprenant plusieurs connecteurs électriques agencés en parallèle.
- le ou les capteur de pression sont configurés pour opérer une ou des mesures de pression de gaz ou de vide (i.e. dépression).
- les liaisons électriques comprennent des fils ou câbles électriques ou des circuits imprimés.
- les liaisons électriques forment des voies.
- le/chaque module de télécommunication peut en outre être configuré pour recevoir une ou des informations provenant d'au moins un équipement de surveillance distant.
- ledit (au moins un) équipement de surveillance distant comprend une Gestion Technique Centralisée (GTC) servant à monitorer à distance le boitier et éventuellement un ou plusieurs autres appareils au sein d'un établissement hospitalier, tel un hôpital, une clinique ou analogue.
- il comprend en outre un écran d'affichage pour y afficher une ou des informations, données, mesures, pictogrammes ou autres éléments graphiques, y compris des caractères alphanumériques.
- les moyens de pilotage sont configurés pour commander le ou les affichages sur l'écran d'affichage.
- l'écran d'affichage est en couleurs ou en noir et blanc.
- l'écran d'affichage peut être à dalle tactile.
- la carte électronique secondaire peut comprendre un ou plusieurs un ou plusieurs (micro)processeur(s) secondaire(s).
- les moyens de télécommunication comprenant la carte électronique secondaire du ou de chaque module de télécommunication comprennent des moyens de transmission pour transmettre (i.e. fournir, émettre ou autre) une ou des informations vers ledit au moins un équipement de surveillance distant.
- la carte électronique principale comprend un unique emplacement à module (i.e. un seul emplacement ou emplacement commun) configuré pour recevoir, c'est-à-dire pour y raccorder, les différents modules de télécommunication amovibles fonctionnant selon des protocoles de communication différents.
- de préférence, le seul emplacement à module, i.e. l'emplacement commun/unique, présent sur la carte électronique principale est configuré pour recevoir, c'est-à-dire pour y raccorder, au moins 2 modules de télécommunication amovibles, c'est-à-dire 2 modules ou plus, de préférence des modules de télécommunication opérant selon des protocoles de communication différents.
- le seul emplacement à module, i.e. l'emplacement commun/unique, présent sur la carte électronique principale est configuré pour recevoir, c'est-à-dire pour y raccorder, au moins :
   - un premier module de télécommunication amovible configuré pour opérer, en particulier pour émettre, selon un premier protocole de communication et/ou alternativement
   - un second module de télécommunication amovible configuré pour opérer, en particulier pour émettre, selon un second protocole de communication.
- le premier protocole de communication est différent du second protocole de communication.
- chaque module de télécommunication est configuré pour être amovible, c'est-à-dire qu'il peut être aisément solidarisé ou désolidarisé (i.e. déconnecté) de la carte principale, par exemple enfiché ou analogue.
- chaque module de télécommunication comprend un système de fixation permettant de solidariser le module de télécommunication à la carte électronique principale, au sein de l'emplacement à module unique, en assurant un raccordement électrique entre la carte électronique secondaire et la carte électronique principale.
- lorsqu'un module de télécommunication est positionné, i.e. solidarisé, enfiché ou inséré, dans l'emplacement à module de la carte électronique principale, la carte électronique secondaire dudit module de télécommunication coopère avec la carte électronique principale de manière à ce que les moyens de pilotage puissent coopérer avec les moyens de transmission pour transmettre une ou des informations à l'équipement de surveillance distant, en particulier en utilisant le protocole de communication du module de télécommunication inséré dans l'emplacement à module de la carte électronique principale.
- les moyens de pilotage comprenant la carte électronique principale commandent les moyens de transmission pour transmettre (i.e. fournir, délivrer, envoyer, émettre ou analogue) une ou des informations soit directement, soit via la carte électronique secondaire.
- l'emplacement à module de la carte électronique principale est configuré pour solidariser des modules de télécommunication de type MODBUS RTU et de type TCP IP.
- le premier module de télécommunication amovible est configuré pour opérer, en particulier pour émettre, en protocole MODBUS RTU en tant que premier protocole de communication.
- le second module de télécommunication amovible est configuré pour opérer, en particulier pour émettre, en protocole TCP IP en tant que second protocole de communication.
- la carte électronique principale comprend en outre des moyens de mémorisation configurés pour mémoriser des données, des mesures et/ou des informations, ou autres.
- les moyens de mémorisation comprennent une mémoire informatique, telle une mémoire flash.
- le boitier comprend des moyens d'alarme commandés (i.e. contrôlés) par les moyens de pilotage.
- les moyens de pilotage du boitier sont configurés pour déclencher une alarme en cas de détection d'une pression excessive ou insuffisante dans l'une ou plusieurs des lignes d'acheminement de fluide.
- les moyens de pilotage sont configurés pour commander un affichage sur le boitier et/ou pour transmettre une information d'alarme audit équipement de télésurveillance distant, typiquement une GTC.
- l'information d'alarme peut être affichée par un afficheur de l'équipement de télésurveillance distant, typiquement une GTC.
- la carte électronique principale comprend les moyens d'alarme commandés (i.e. contrôlés) par les moyens de pilotage.
- les moyens de pilotage contrôlent les moyens d'alarme pour déclencher une alarme en cas de détection d'une pression inadéquate, telle une surpression (i.e. pression excessive) ou une sous-pression (i.e. pression insuffisante) par rapport à un ou des seuils de pression mémorisés.
- les moyens d'alarme sont sonores ou visuels, ou les deux.
- les moyens d'alarme comprennent une ou plusieurs LED ou analogue.
- les moyens d'alarme comprennent un système buzzer ou analogue.
- les moyens d'alarme comprennent un affichage d'une information d'alarme sur l'écran d'affichage.
- les moyens de pilotage sont configurés pour commander un affichage d'une alarme (ou alerte) visuelle sur l'écran d'affichage, en réponse à un déclenchement d'alarme.
- selon un mode de réalisation, le (chaque) module de télécommunication, en particulier la carte électronique secondaire, comprend des moyens de raccordement de réseau configurés pour permettre le raccordement à un réseau de communication reliant le boitier à l'équipement de surveillance distant de manière à assurer l'émission (i.e. transfert) d'informations du boitier vers l'équipement de surveillance distant, par exemple une CTG.
- les moyens de raccordement de réseau comprennent au moins un connecteur de raccordement de réseau.
- le réseau de communication est un réseau de communication filaire ou binaire.
- l'équipement de surveillance distant est relié au boitier par le réseau de communication, en particulier par une ou des liaisons filaires, tel que fil conducteur ou fibre optique.
- selon un autre mode de réalisation, la carte électronique secondaire comprend un système d'émission (i.e. d'envoi ou de transfert) d'informations sans fil, par un système d'émission en mode ondes radio, wifi, bluetooth^{®} , Lora^{®} ou autre. Ce système d'émission d'informations sans fil comprend une antenne émettrice.
- l'équipement de surveillance distant, par exemple une CTG, comprend un système de bus communiquant avec le boitier de surveillance.
- l'équipement de surveillance distant est situé dans l'établissement hospitalier, de préférence dans un autre local ou site que celui où se trouve le boitier.
- alternativement, l'équipement de surveillance distant est situé sur un autre site distant, par exemple chez un fournisseur de gaz.
- le système de bus est configuré pour recueillir une ou des informations émises par le boitier.
- le système de bus est configuré pour coopérer avec le boitier via le réseau de communication relié aux moyens de raccordement de réseau de la carte électronique secondaire.
- il comprend en outre un coffret au sein duquel sont agencés la carte électronique principale et les moyens de pilotage.
- il comprend en outre des moyens d'alimentation en courant électrique, en particulier un ou des connecteurs destinés à être reliés au secteur électrique (110/220 V).
- les moyens d'alimentation en courant électrique sont configurés pour alimenter -directement ou indirectement- en courant électrique tous les composants ayant besoin de courant électrique pour fonctionner, notamment la carte électronique principale, les moyens de pilotage, l'écran d'affichage...
- les moyens de pilotage sont configurés pour traiter les mesures de pression provenant des capteurs et détecter une pression excessive ou insuffisante dans l'une ou plusieurs des lignes d'acheminement de fluide.
- les moyens de pilotage sont configurés pour comparer les mesures de pression provenant des différentes liaisons électriques, aussi appelées voies de surveillance, reliées aux capteurs, avec les seuils de pression basse et/ou de pression haute des paramètres de configuration associés aux différentes voies de surveillance et mémorisés par les moyens de mémorisation de données afin de déclencher une alarme sonore et/ou visuelle lorsqu'une pression excessive ou insuffisante est détectée.
- les moyens de pilotage sont configurés pour déterminer qu'une pression mesurée correspond à une pression excessive lorsque ladite pression mesurée est supérieure à un seuil de pression haute donné, i.e. mémorisé.
- les moyens de pilotage sont configurés pour déterminer qu'une pression mesurée correspond à une pression insuffisante lorsque ladite pression mesurée est inférieure à un seuil de pression basse donné, i.e. mémorisé.
- il comprend de 2 à 10 liaisons électriques, appelées voies de surveillance, de préférence de 3 à 8 voies de surveillance, par exemple 4 voies.
- les voies de surveillance sont agencées en parallèle les unes des autres.
- le boitier est programmable, c'est-à-dire paramétrable.
- le boitier est programmable à l'aide de paramètres de configuration mémorisés.
- les paramètres de configuration du boitier comprennent un type de fluide choisi parmi l'oxygène, l'air, le N₂O, un mélange N₂O/O₂ ou le vide, de préférence l'oxygène, l'air ou le vide.
- les paramètres de configuration du boitier comprennent pour un gaz sous pression, un seuil de pression basse compris entre 3 et 7 bar abs et un seuil de pression haute compris entre 4.5 et 10 bar abs, et/ou pour du vide (dépression), un seuil de pression basse compris entre -0.8 et -0.3 bar abs et un seuil de pression haute compris entre -0.1 et 0.5 bar abs.
- les paramètres de configuration du boitier comprennent un type de capteur choisi parmi un capteur 0 à 16 bar, un capteur 0 à 250 bars et un capteur 0 à -0,90 bar.
- alternativement, les paramètres de configuration du boitier comprennent un type de capteur, y compris des capteurs tout ou rien.
- les paramètres de configuration du boitier comprennent, pour une source de production de gaz, un seuil de pression basse compris entre 15 et 25 bar abs, par exemple de l'ordre de 17 bar abs, ou un seuil de pression haute compris entre 110 et 150 bar abs, par exemple de l'ordre de 130 bar abs.
- l'emplacement à module de la carte électronique principale est configuré pour rester libre sans module de télécommunication inséré, lorsque aucun protocole de communication n'est souhaité ou nécessaire.
- le boitier de surveillance est configuré pour fonctionner normalement en l'absence de module de télécommunication inséré dans l'emplacement à module de la carte électronique principale, mais sans émettre alors de données.
- le ou les capteurs de pression sont agencés sur une ou des lignes d'acheminement de fluide choisies parmi une ligne d'acheminement d'oxygène, une ligne d'acheminement d'air, une ligne d'acheminement de N₂O ou de mélange N₂O/O₂ et/ou une ligne d'acheminement de vide (i.e. dépression).
- le boitier de surveillance de fluides est fixé à une (ou des) paroi, c'est-à-dire un mur, une cloison ou analogue, de l'établissement hospitalier.
- la ou les lignes d'acheminement de fluide comprennent des canalisations, conduites ou lignes de gaz ou de vide.
- la ou les lignes d'acheminement de fluide sont fixées à une (ou des) paroi, c'est-à-dire un mur, une cloison, et/ou à un (ou des) plafond ou analogue, de l'établissement hospitalier.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'acheminement de fluide comprenant un boitier de surveillance selon l'invention,
Fig. 2 schématise un mode de réalisation de la face avant d'un boitier pour une installation d'acheminement de fluide selon l'invention, et
Fig. 3 schématise le raccordement de différents modules de télécommunication dans l'emplacement à module dédié d'un boitier de surveillance d'une installation d'acheminement de fluide selon l'invention.

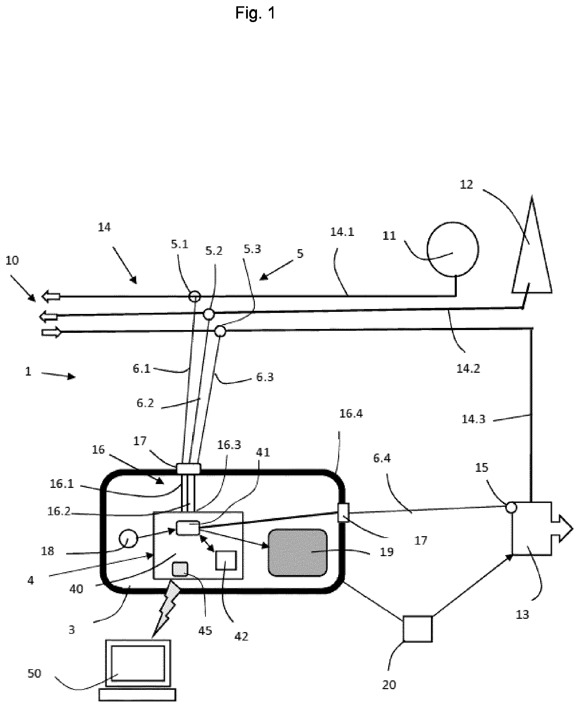

Fig. 1 schématise un mode de réalisation d'une installation 1 d'acheminement de fluide selon l'invention comprenant plusieurs lignes d'acheminement de fluide 14.1-14.3 faisant partie d'un réseau de canalisations 14 de fluides agencé au sein d'un établissement hospitalier 60, tel un hôpital, une clinique ou analogue. Par exemple, une ligne d'acheminement d'oxygène 14.1, une ligne d'acheminement d'air médical 14.2 et une ligne d'acheminement de vide 14.3, c'est-à-dire de dépression (i.e. < 1 atm).

Le réseau 14 de canalisations permet d'acheminer les fluides médicaux, i.e. ici des gaz et du vide (dépression), depuis leur site de production ou de stockage sur le site de l'établissement hospitalier 60, jusqu'à des prises de distribution murales 10 agencées dans leurs lieux d'utilisation au sein de l'établissement hospitalier 60, telles les salles de soins, d'opération ou de réveil, les urgences, les chambres ou autres.

Ainsi, l'air de qualité médicale peut être produit directement sur site au moyen d'une unité de type PSA air 12, comme décrit par EP-A-864818, et il en va de même du vide qui peut aussi être produit sur site au moyen d'une (ou plusieurs) pompe à vide 13, comme décrit par EP-A-1026567. Par ailleurs, l'oxygène ou le protoxyde d'azote (N₂O) peuvent être produits hors site, avant d'y être amenés par camion-citerne ou analogue, où ils sont stockés dans des réservoirs de stockage 11, ou des bouteilles de gaz qui peuvent être agencées en cadre réunissant plusieurs bouteilles reliées entre elles. Alternativement, l'oxygène peut aussi être produit sur site par unité de type VSA O₂. De façon connue, une unité PSA (pressure swing adsorption) permet de produire du gaz par modulation de pression, alors qu'une unité VSA (vacuum swing adsorption) permet de produire du gaz par modulation de vide.

Les lignes d'acheminement de fluide 14.1-14.3 du réseau de canalisations 14 sont habituellement fixées sur les murs ou parois de l'établissement hospitalier. Il en va de même des prises murales 10 situées aux extrémités libres de ces lignes d'acheminement de fluide, comme décrit par EP-A-3719377.

L'installation 1 comprend par ailleurs un boitier de surveillance 3 du réseau 14 comprenant des moyens de pilotage 4 comprenant une carte électronique principale 40 sur laquelle est agencé un (ou plusieurs) (micro) processeur(s) 41 mettant en œuvre un ou des algorithmes, et configurés notamment pour traiter des mesures, informations, données, ou autres, et/ou déclencher une ou des alarmes.

Les moyens de pilotage 4 peuvent aussi être appelés unité de pilotage, unité ou moyens de traitement d'information, électronique embarquée ou analogue. Les moyens de pilotage 4 comprennent avantageusement des moyens de mémorisation 42, en particulier agencés sur la carte électronique principale 40, servant à mémoriser ou stocker des paramètres de configuration, des données ou d'autres informations, telle une mémoire flash ou analogue.

Le boitier ou appareil de surveillance 3 de l'installation d'acheminement de fluide 1 comprend un coffret périphérique qui est lui-aussi fixé à un mur ou une paroi de l'établissement hospitalier 60, lequel embarque les moyens de pilotage 4, en particulier la carte électronique principale 40,

Le boitier 3 comprend par ailleurs des moyens de raccordement électrique 17 comprenant un ensemble de connecteurs de raccordement électrique, tel un bornier électrique ou analogue, relié à plusieurs voies 16 ou liaisons électriques, raccordées aux moyens de mesure ou capteurs 5, typiquement des capteurs de pression 5.1-5.3 de manière à fournir les mesures de pression mesurées ou déterminée par ces moyens de mesure 5, aux moyens de pilotage 4 qui les traitent. Avantageusement, on peut aussi prévoir un dispositif de surveillance 15 de la production de vide par la pompe à vide 13 ou par l'une des autres sources de gaz 11, 12, i.e. PSA ou autre.

Plus précisément, les capteurs de pression 5.1-5.3 faisant office de moyens de mesure 5 sont agencés sur les différentes lignes d'acheminement de fluide 14.1-14.3 du réseau 14 de manière à pouvoir mesurer la pression des fluides circulant dans ces différentes lignes 14.1-14.3. Ces capteurs de pression 5.1-5.3 sont reliés électriquement aux moyens de pilotage 4 via les liaisons 6 électriques, tels des câbles électriques ou analogue, et les moyens de raccordement électrique 17, tels un ensemble de connecteurs électriques, par exemple un bornier ou analogue, comprenant des emplacements de raccordement, et les différentes voies 16 ; 16.1-16.4, afin de leur fournir les mesures de pression opérées, i.e. valeurs ou signal. A titre d'exemple, on peut utiliser un capteur de pression analogique 0-16 bars de marque Huba^{®}.

De façon analogue, la pompe à vide 13 servant à produire le vide, c'est-à-dire la dépression servant à l'aspiration, peut être équipée d'un dispositif de surveillance 15, qui renvoie une synthèse des défauts de l'équipement (i.e. pannes ou anomalies), relié, lui aussi, aux moyens de pilotage 4, via une voie dédiée 6.4, pour leur fournir des informations de fonctionnement de la pompe 13 afin de pouvoir détecter tout dysfonctionnement de cette pompe à vide 13 et déclencher une (ou des) alarme en réponse à cette détection.

Les mesures de pression provenant des moyens de mesure 5 sont traitées informatiquement par les moyens de pilotage 4, en particulier par le (ou les) processeur(s) 41 de la carte électronique principale 40, pour détecter tout problème de pression ou dysfonctionnement éventuel du réseau 14, c'est-à-dire des lignes d'acheminement de fluide 14.1-14.3, ou de la pompe à vide 13, par exemple des pressions excessives ou insuffisantes, et déclencher ensuite une (des) alarme sonore ou visuelle en réponse à la détection par exemple d'une chute de pression dans l'une des conduites de gaz du réseau 14 ou un dysfonctionnement de la pompe à vide 13, afin d'alerter le personnel soignant et lui permettre de prendre des mesures appropriées.

Des moyens d'alimentation électrique 20, tel que le secteur (110/220 V), alimentent électriquement les différents composants de l'installation 1 ayant besoin de courant électrique pour fonctionner, en particulier les moyens de pilotage 4, les capteurs 5, le dispositif de surveillance 15, la pompe à vide 13....

Afin que les moyens de pilotage 4 puissent remplir leur fonction, le boitier de surveillance 3 est programmé ou paramétré, lors de sa mise en service notamment, afin d'assurer une conformité de l'installation, en particulier en termes de sécurité. Ainsi, les seuils de pression d'alarme, c'est-à-dire les seuils de pression haute et basse, doivent être définis avec précision, ainsi que d'autres paramètres de configuration. Pour ce faire, chaque voie 16 du boitier 3 est paramétrée au sein du boitier de surveillance 3, c'est-à-dire que des paramètres de configuration spécifiques associés à chacune des différentes voies 16 sont mémorisés au sein des moyens de mémorisation 4-2.

Le paramétrage, c'est-à-dire les paramètres de configuration, des différentes voies 16 du boîtier de surveillance 3 va varier selon le fluide surveillé par chacune de ces voies 16 et/ou, éventuellement selon le type d'utilisation, par exemple surveillance de la production ou surveillance de l'alimentation d'un service. En général, un boîtier de surveillance 3 peut comprendre jusqu'à 8 à 10 voies différentes.

Les paramètres de configuration sont par exemple : des types de fluides, typiquement oxygène, air, vide, protoxyde d'azote (N₂O)... ; des seuils haut et/ou bas de pression d'alarme correspond au type de fluide considéré, des types de capteurs utilisés, les services hospitaliers alimentés par les fluide considérés, par exemple cardiologie...

L'opérateur peut réaliser les sélections via des moyens de sélection 18, tel qu'un ou des boutons, touches ou analogue, aménagées sur ou dans le boitier 3 ou, dans le cas où les moyens d'affichage 19 comprennent un écran d'affichage à dalle tactile, qui affichent les sélections, les paramètres et d'une façon générale, toutes les informations et autres données utiles à l'opérateur.

D'une façon générale, les moyens d'affichage 19 sont pilotés par les moyens de pilotage 4, en particulier la carte électronique principale 40 à microprocesseur 41 qui porte aussi préférentiellement les moyens de mémorisation 42, telle une mémoire flash.

Les liaisons électriques 6.1-6.3 reliant le boitier 3 aux capteurs 5 sont typiquement des câbles électriques ou analogues permettant d'acheminer les signaux de mesure, qu'ils soient analogiques ou numériques. De même, la quatrième voie 6.4 est définie par un ensemble de paramètres de configuration spécifiques permettant une surveillance de production via un capteur de fonctionnement de la pompe à vide.

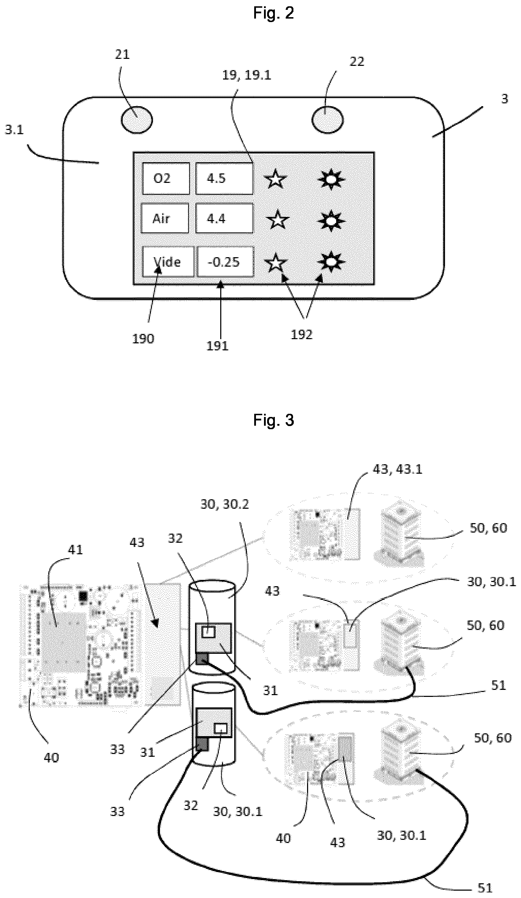

Fig. 2 représente un schéma d'un mode de réalisation de la face avant 3.1 d'un boitier 3 de surveillance selon l'invention, par exemple à coque ou carcasse rigide en polymère. On voit qu'elle porte les moyens d'affichage 19, à savoir ici un écran 19.1 de visualisation d'informations, de préférence en couleurs.

L'écran 19.1 affiche ici différentes informations, telles que :
- le nom du fluide 190 de chaque voie, e.g. ici oxygène, air, vide...
- la valeur de (dé)pression 191 mesurée pour chaque fluide (en bar),
- un ou des pictogrammes 192 illustrant un état des différentes voies, en particulier pression « normale » ou « alarme » en cas de pression excessive ou insuffisante.

Par ailleurs, le boitier 3 porte aussi ici, sur sa face avant 3.1, un bouton d'acquittement d'alarme 22 et un bouton de test 21 servant à vérifier le bon fonctionnement des moyens d'alerte, notamment du buzzer sonore ou des pictogrammes 192.

Le boitier de surveillance 1 communique avec un équipement de télésurveillance distant 50, typiquement une Gestion Technique Centralisée (GTC) servant classiquement à monitorer à distance plusieurs appareils au sein de l'établissement hospitalier 60, à savoir non seulement le boîtier de surveillance 1 des fluides médicaux mais aussi des boitiers d'alarme incendie, le système de ventilation, le système de chauffage et/ou climatisation... de l'hôpital ou analogue.

Le boitier de surveillance 1 communique avec un équipement de télésurveillance distant 50, typiquement un centre technique de surveillance des gaz médicaux, localisé par exemple chez un fournisseur de gaz, servant classiquement à indiquer à ce dernier les réserves de gaz médical restant et à déclencher dès réapprovisionnement de l'établissement hospitalier.

Lorsqu'une alarme se déclenche au niveau du boitier 3, l'information ou l'alerte correspondante peut être simplement affichée sur le boitier 3 mai aussi transmise à l'équipement de télésurveillance distant 50, typiquement une GTC.

Pour ce faire, le boîtier de surveillance 1 est équipé de moyens de télécommunication 45 mettant en œuvre un ou des protocoles de communication différents.

Selon l'invention, afin de pouvoir répondre aux différentes configurations hospitalières, le boitier 3 de l'installation d'acheminement de fluide 1 selon l'invention comprend agencé sur la carte électronique principale 40, au moins un emplacement à module 43 qui permet de raccorder différents modules de télécommunication amovibles 30 ; 30.1, 30.2, c'est-à-dire détachables, comprenant chacun (au moins) une carte électronique secondaire 31 portant des moyens de télécommunication 32 permettant de transmettre, c'est-à-dire envoyer, émettre, produire, délivrer, fournir ou analogue, des informations (i.e., données, mesures, information d'alarme...) vers un équipement de surveillance distant 60, typiquement une GTC ou analogue.

Comme illustré en Fig. 3, pouvoir raccorder différents modules de télécommunication amovibles 30 ; 30.1, 30.2, c'est-à-dire fonctionnant selon des protocoles de communication différents, à l'unique emplacement à module 43 de la carte électronique principale 40 du boitier 3 permet de choisir le protocole de communication le plus adapté, donc de pouvoir choisir et mettre en œuvre différents protocoles de communication en fonction de la configuration particulière de l'établissement hospitalier 60 considéré.

Chaque module de télécommunication 30 est amovible, c'est-à-dire qu'il est configuré pour pouvoir être fixé ou, à l'inverse, retiré aisément de l'emplacement à module 43 commun, c'est-à-dire unique, de la carte électronique principale 40 du boitier 3, par exemple le module de télécommunication 30 peut être enfiché dans la carte électronique principale 40 ou analogue. Autrement dit, le boitier 3 est modulaire et peut accueillir des modules de télécommunication 30 différents, c'est-à-dire fonctionnant selon des protocoles de communication distincts.

Ainsi, comme illustré en Fig. 3, en fixant le module de télécommunication 30 ; 30.1, 30.2 le plus adapté à chaque situation particulière, au sein de l'emplacement à module 43 de la carte électronique principale 40 du boitier 1, on peut mettre en œuvre un protocole de communication MODBUS RTU grâce à un module dédié 30.1 de type MODBUS RTU ou un protocole de communication TCP IP grâce à un module 30.2 de type TCP IP. Passer d'un protocole à un autre peut dès lors se faire aisément en découplant l'un des modules 30.1 pour y insérer l'autre module 30.2, ou inversement.

Toutefois, si aucun protocole de communication n'est souhaité ou nécessaire, l'emplacement à module 43 de la carte électronique principale 40 du boitier 1 peut rester libre 43.1, c'est-à-dire sans module inséré. Dans ce cas, le boitier de surveillance 3 peut continuer à fonctionner normalement mais n'émet pas de données ou analogue.

Une fois raccordé à l'emplacement à module 43, le module de télécommunication 30 ; 30.1, 30.2 coopère, via sa carte électronique secondaire 31 qui comprend typiquement un (des) microprocesseur secondaire, avec les moyens de pilotage 4 du boitier 3, en particulier la carte électronique principale 4, pour transmettre des informations (i.e. données ou autres) vers l'équipement de surveillance distant 50, telle une GTC située dans le bâtiment hospitalier 60.

Pour ce faire, les moyens de télécommunication 32 de la carte électronique secondaire 31 du module de télécommunication 30 comprennent des moyens de transmission pour transmettre, émettre, délivrer, envoyer, fournir ou similaire, des informations (i.e. données, alarme...) vers l'équipement de surveillance distant 50.

En particulier, les moyens de transmission du module de télécommunication 30, en particulier la carte électronique secondaire 31, comprennent des moyens de raccordement de réseau 33 auxquels on vient raccorder un réseau de communication 51 filaire reliant le boitier 3 à l'équipement de surveillance distant 50, typiquement un (des) connecteur de raccordement de réseau. Le réseau de communication 51 filaire comprend par exemple des liaisons électriques ou optique, tels que câbles, fibres optiques ou autres, servant à acheminer les données ou autres informations jusqu'à l'équipement de surveillance distant 50, telle une GTC située dans le bâtiment hospitalier 60.

Selon un mode de réalisation, les échanges d'informations peuvent se faire via un système de bus pouvant interroger le boitier 3 et récupérer ensuite les informations émises par le boitier 3 en réponse à l'interrogation.

Ainsi, lorsque les moyens de pilotage 4, en particulier le microprocesseur 41 de la carte électronique principale 40, détectent une pression inadéquate, telle une surpression (i.e. pression excessive) ou une sous-pression (i.e. pression insuffisante) par rapport à un ou des seuils de pression mémorisés au sein des moyens de mémorisation 42, ils commandent des moyens d'alarme sonores ou visuels qui peuvent être agencés sur la carte électronique principale 40, tel un buzzer sonore, et/ou ailleurs sur le boitier 3, comme une ou plusieurs LEDs ou analogue, ou se présentant sous forme d'un affichage d'alarme sur l'écran d'affichage 19, afin d'alerter un opérateur qu'un capteur 5 a mesuré un défaut de pression.

En parallèle, cette information d'alarme va être communiquée à l'équipement de surveillance distant 50, telle une GTC du bâtiment hospitalier 60 via les moyens de télécommunication 32 de la carte électronique secondaire 31 du module de télécommunication 30, comme expliqué ci-avant, et via le réseau de communication 51 filaire reliant le boitier 3 à l'équipement de surveillance distant 50.

Ceci permet de recueillir cette information d'alarme à distance et de prendre alors des mesures adéquates pour remédier au problème de pression mesuré.

L'installation 1 comprenant un boitier de surveillance de fluide 3 selon l'invention est particulièrement adaptée à une utilisation dans un établissement hospitalier 60 pour y surveiller le réseau de fluides, i.e. gaz et/ou vide, qui est installé au sein de l'établissement hospitalier 60, notamment sur les murs ou les plafonds, grâce à des capteurs de pression 5 agencés sur les lignes d'acheminement de fluide 14 dudit réseau de fluides, lesquels sont relié électriquement au boitier de surveillance de fluide 3 selon l'invention, en particulier pour surveiller une ligne d'acheminement d'oxygène, une ligne d'acheminement d'air, une ligne d'acheminement de N₂O ou de mélange N₂O/O₂ et/ou une ligne d'acheminement de vide.

## Revendications

1. Installation d'acheminement de fluide (1) dans un établissement hospitalier (60), comprenant un boitier de surveillance de fluide (3) relié électriquement à un ou des capteurs de pression (5), agencés sur une ou des lignes d'acheminement de fluide (14), ledit boitier de surveillance de fluide (3) comprenant :
- des moyens de pilotage (4) comprenant une carte électronique principale (40) comprenant au moins un processeur (41),
- des moyens de raccordement électrique (17) configurés pour y raccorder électriquement au moins un capteur de pression (5) configuré pour opérer une ou des mesures de pression de fluide, et
- des liaisons électriques (16) pour raccorder électriquement les moyens de raccordement électrique (17) aux moyens de pilotage (4) de manière à fournir auxdits moyens de pilotage (4), la ou les mesures de pression provenant du ou des capteur de pression (5),
**caractérisée en ce que** la carte électronique principale (40) du boitier de surveillance de fluide (3) comprend un seul emplacement à module (43) configuré pour permettre d'y raccorder différents modules de télécommunication (30 ; 30.1, 30.2) amovibles, chaque module de télécommunication (30) amovible comprenant au moins une carte électronique secondaire (31) portant des moyens de télécommunication (32), chaque module de télécommunication (30) amovible étant configuré pour coopérer, via la carte électronique secondaire (31), avec les moyens de pilotage (4) comprenant la carte électronique principale (40) pour transmettre une ou des informations vers au moins un équipement de surveillance distant (50).

2. Installation selon la revendication 1, **caractérisée en ce que** les moyens de télécommunication (32) comprennent des moyens de transmission pour transmettre une ou des informations vers ledit au moins un équipement de surveillance distant (50).

3. Installation selon la revendication 1, **caractérisée en ce que** l'emplacement à module (43) de la carte électronique principale (40) est configuré pour rester libre (43.1) sans module de télécommunication (30 ; 30.1, 30.2) inséré, lorsque aucun protocole de communication n'est souhaité ou nécessaire.

4. Installation selon la revendication 2, **caractérisée en ce que** lorsqu'un module de télécommunication (30) est positionné dans l'emplacement à module (43) de la carte électronique principale (40), la carte électronique secondaire (31) dudit module de télécommunication (30) coopère avec la carte électronique principale (40) de manière à ce que les moyens de pilotage (4) puissent coopérer avec les moyens de transmission pour transmettre une ou des informations à l'équipement de surveillance distant (50).

5. Installation selon l'une des revendications, **caractérisée en ce que** le seul emplacement à module (43) présent sur la carte électronique principale (40) est configuré pour recevoir au moins :
- un premier module de télécommunication amovible configuré pour opérer selon un premier protocole de communication et/ou
- un second module de télécommunication amovible configuré pour opérer selon un second protocole de communication différent du premier protocole de communication.

6. Installation selon l'une des revendications 3 ou 5, **caractérisée en ce que** l'emplacement à module (43) de la carte électronique principale (40) est configuré pour solidariser des modules de télécommunication (30 ; 30.1, 30.2) de type MODBUS RTU et de type TCP IP.

7. Installation selon l'une des revendications 3, 5 ou 6, **caractérisée en ce que** le premier protocole de communication est le protocole MODBUS RTU et le second protocole de communication est le protocole TCP IP.

8. Installation selon la revendication 1, **caractérisée en ce que** la carte électronique principale (40) comprend en outre des moyens de mémorisation (42).

9. Installation selon la revendication 1, **caractérisée en ce que** la carte électronique principale (40) comprend en outre des moyens d'alarme commandés par les moyens de pilotage (3).

10. Installation selon la revendication 1, **caractérisée en ce que** le module de télécommunication (30), en particulier la carte électronique secondaire (31), comprend des moyens de raccordement de réseau (33) configurés pour permettre d'y raccordement un réseau de communication (51) reliant le boitier (3) à l'équipement de surveillance distant (50).

11. Installation selon la revendication 10, **caractérisé en ce que** les moyens de raccordement de réseau (33) comprennent au moins un connecteur de raccordement de réseau.

12. Installation selon la revendication 1, **caractérisée en ce que** la ou les lignes d'acheminement de fluide (14) comprennent une ligne d'acheminement d'oxygène, une ligne d'acheminement d'air, une ligne d'acheminement de N₂O ou de mélange N₂O/O₂, et/ou une ligne d'acheminement de vide.

13. Installation selon l'une des revendications, **caractérisée en ce que** les moyens de pilotage (4) du boitier (3) sont configurés pour traiter les mesures de pression provenant des capteurs (5) et détecter une pression excessive ou insuffisante dans l'une ou plusieurs des lignes d'acheminement de fluide (14).

14. Installation selon la revendication 13, **caractérisée en ce que** les moyens de pilotage (4) du boitier (3) sont configurés pour déclencher une alarme en cas de détection d'une pression excessive ou insuffisante dans l'une ou plusieurs des lignes d'acheminement de fluide (14).

15. Installation selon les revendications 1 et 14, **caractérisée en ce que** les moyens de pilotage (4) sont configurés pour commander un affichage sur le boitier (3) et/ou pour transmettre une information d'alarme audit équipement de télésurveillance distant (50).

## Patentansprüche

1. Anlage zur Fluidförderung (1) in einer Krankenhauseinrichtung (60), umfassend ein Fluidüberwachungsgehäuse (3), das elektrisch mit einem oder mehreren Drucksensoren (5) verbunden ist, die an einer oder mehreren Fluidförderleitungen (14) angeordnet sind, wobei das besagte Fluidüberwachungsgehäuse (3) Folgendes umfasst:
- Steuermittel (4), die eine elektronische Hauptplatine (40) umfassen, die mindestens einen Prozessor (41) umfasst,
- elektrische Anschlussmittel (17), die konfiguriert sind, um daran mindestens einen Drucksensor (5) elektrisch anzuschließen, der konfiguriert ist, um eine oder mehrere Fluiddruckmessungen durchzuführen, und
- elektrische Verbindungen (16), um die elektrischen Anschlussmittel (17) elektrisch mit den Steuermitteln (4) zu verbinden, um den besagten Steuermitteln (4) die eine oder die mehreren Druckmessungen, die von dem einen oder den mehreren Drucksensoren (5) stammen, bereitzustellen,
**dadurch gekennzeichnet, dass** die elektronische Hauptplatine (40) des Fluidüberwachungsgehäuses (3) einen einzigen Modulsteckplatz (43) umfasst, der konfiguriert ist, um den Anschluss verschiedener abnehmbarer Telekommunikationsmodule (30; 30.1, 30.2) zu ermöglichen, wobei jedes abnehmbare Telekommunikationsmodul (30) mindestens eine elektronische Sekundärplatine (31) umfasst, die Telekommunikationsmittel (32) trägt, wobei jedes abnehmbare Telekommunikationsmodul (30) konfiguriert ist, um über die elektronische Sekundärplatine (31) mit den Steuermitteln (4), die die elektronische Hauptplatine (40) umfassen, zusammenzuwirken, um eine oder mehrere Informationen an mindestens ein Fernüberwachungsgerät (50) zu übertragen.

2. Anlage nach Anspruch 1, **dadurch gekennzeichnet, dass** die Telekommunikationsmittel (32) Übertragungsmittel zum Übertragen einer oder mehrerer Informationen an das besagte mindestens eine Fernüberwachungsgerät (50) umfassen.

3. Anlage nach Anspruch 1, **dadurch gekennzeichnet, dass** der Modulsteckplatz (43) der elektronischen Hauptplatine (40) konfiguriert ist, um frei (43.1) zu bleiben, ohne eingesetztes Telekommunikationsmodul (30; 30.1, 30.2), wenn kein Kommunikationsprotokoll gewünscht oder erforderlich ist.

4. Anlage nach Anspruch 2, **dadurch gekennzeichnet, dass**, wenn ein Telekommunikationsmodul (30) in dem Modulsteckplatz (43) der elektronischen Hauptplatine (40) positioniert ist, die elektronische Sekundärplatine (31) des besagten Telekommunikationsmoduls (30) mit der elektronischen Hauptplatine (40) so zusammenwirkt, dass die Steuermittel (4) mit den Übertragungsmitteln zusammenwirken können, um eine oder mehrere Informationen an das Fernüberwachungsgerät (50) zu übertragen.

5. Anlage nach einem der vorhergehenden Ansprüche, **dadurch gekennzeichnet, dass** der einzige Modulsteckplatz (43), der auf der elektronischen Hauptplatine (40) vorhanden ist, konfiguriert ist, um mindestens aufzunehmen:
- ein erstes abnehmbares Telekommunikationsmodul, das konfiguriert ist, um gemäß einem ersten Kommunikationsprotokoll zu arbeiten und/oder
- ein zweites abnehmbares Telekommunikationsmodul, das konfiguriert ist, um gemäß einem zweiten Kommunikationsprotokoll zu arbeiten, das sich von dem ersten Kommunikationsprotokoll unterscheidet.

6. Anlage nach Anspruch 3 oder 5, **dadurch gekennzeichnet, dass** der Modulsteckplatz (43) der elektronischen Hauptplatine (40) konfiguriert ist, um Telekommunikationsmodule (30; 30.1, 30.2) vom Typ MODBUS RTU und vom Typ TCP IP zu befestigen.

7. Anlage nach einem der Ansprüche 3, 5 oder 6, **dadurch gekennzeichnet, dass** das erste Kommunikationsprotokoll das MODBUS RTU-Protokoll ist und das zweite Kommunikationsprotokoll das TCP IP-Protokoll ist.

8. Anlage nach Anspruch 1, **dadurch gekennzeichnet, dass** die elektronische Hauptplatine (40) außerdem Speichermittel (42) umfasst.

9. Anlage nach Anspruch 1, **dadurch gekennzeichnet, dass** die elektronische Hauptplatine (40) außerdem von den Steuermitteln (4) gesteuerte Alarmmittel umfasst.

10. Anlage nach Anspruch 1, **dadurch gekennzeichnet, dass** das Telekommunikationsmodul (30), insbesondere die elektronische Sekundärplatine (31), Netzwerkanschlussmittel (33) umfasst, die konfiguriert sind, um den Anschluss eines Kommunikationsnetzwerks (51) zu ermöglichen, das das Gehäuse (3) mit dem Fernüberwachungsgerät (50) verbindet.

11. Anlage nach Anspruch 10, **dadurch gekennzeichnet, dass** die Netzwerkanschlussmittel (33) mindestens einen Netzwerkanschlussstecker umfassen.

12. Anlage nach Anspruch 1, **dadurch gekennzeichnet, dass** die eine oder die mehreren Fluidförderleitungen (14) eine Sauerstoffförderleitung, eine Luftförderleitung, eine N2O- oder N2O/O2-Gemisch-Förderleitung und/oder eine Vakuumförderleitung umfassen.

13. Anlage nach einem der vorhergehenden Ansprüche, **dadurch gekennzeichnet, dass** die Steuermittel (4) des Gehäuses (3) konfiguriert sind, um die Druckmessungen von den Sensoren (5) zu verarbeiten und einen übermäßigen oder unzureichenden Druck in einer oder mehreren der Fluidförderleitungen (14) zu erkennen.

14. Anlage nach Anspruch 13, **dadurch gekennzeichnet, dass** die Steuermittel (4) des Gehäuses (3) konfiguriert sind, um im Falle der Erkennung eines übermäßigen oder unzureichenden Drucks in einer oder mehreren der Fluidförderleitungen (14) einen Alarm auszulösen.

15. Anlage nach den Ansprüchen 1 und 14, **dadurch gekennzeichnet, dass** die Steuermittel (4) konfiguriert sind, um eine Anzeige am Gehäuse (3) zu steuern und/oder eine Alarminformation an das besagte Fernüberwachungsgerät (50) zu übertragen.

## Claims

1. A fluid supply installation (1) in a hospital establishment (60), comprising a fluid monitoring box (3) electrically connected to one or more pressure sensors (5), arranged on one or more fluid supply lines (14), said fluid monitoring box (3) comprising:
- control means (4) comprising a main electronic board (40) comprising at least one processor (41),
- electrical connection means (17) configured to electrically connect thereto at least one pressure sensor (5) configured to perform one or more fluid pressure measurements, and
- electrical links (16) for electrically connecting the electrical connection means (17) to the control means (4) so as to provide said control means (4) with the one or more pressure measurements originating from the one or more pressure sensors (5),
**characterized in that** the main electronic board (40) of the fluid monitoring box (3) comprises a single module slot (43) configured to allow for the connection of different removable telecommunication modules (30; 30.1, 30.2), each removable telecommunication module (30) comprising at least one secondary electronic board (31) carrying telecommunication means (32), each removable telecommunication module (30) being configured to cooperate, via the secondary electronic board (31), with the control means (4) comprising the main electronic board (40) to transmit one or more pieces of information to at least one remote monitoring equipment (50).

2. The installation according to claim 1, **characterized in that** the telecommunication means (32) comprise transmission means for transmitting one or more pieces of information to said at least one remote monitoring equipment (50).

3. The installation according to claim 1, **characterized in that** the module slot (43) of the main electronic board (40) is configured to remain free (43.1) without an inserted telecommunication module (30; 30.1, 30.2) when no communication protocol is desired or necessary.

4. The installation according to claim 2, **characterized in that** when a telecommunication module (30) is positioned in the module slot (43) of the main electronic board (40), the secondary electronic board (31) of said telecommunication module (30) cooperates with the main electronic board (40) such that the control means (4) can cooperate with the transmission means to transmit one or more pieces of information to the remote monitoring equipment (50).

5. The installation according to any one of the preceding claims, **characterized in that** the single module slot (43) present on the main electronic board (40) is configured to receive at least:
- a first removable telecommunication module configured to operate according to a first communication protocol and/or
- a second removable telecommunication module configured to operate according to a second communication protocol different from the first communication protocol.

6. The installation according to claim 3 or 5, **characterized in that** the module slot (43) of the main electronic board (40) is configured to secure telecommunication modules (30; 30.1, 30.2) of MODBUS RTU type and TCP IP type.

7. The installation according to any one of claims 3, 5 or 6, **characterized in that** the first communication protocol is the MODBUS RTU protocol and the second communication protocol is the TCP IP protocol.

8. The installation according to claim 1, **characterized in that** the main electronic board (40) further comprises memory means (42).

9. The installation according to claim 1, **characterized in that** the main electronic board (40) further comprises alarm means controlled by the control means (4).

10. The installation according to claim 1, **characterized in that** the telecommunication module (30), in particular the secondary electronic board (31), comprises network connection means (33) configured to allow for the connection of a communication network (51) linking the box (3) to the remote monitoring equipment (50).

11. The installation according to claim 10, **characterized in that** the network connection means (33) comprise at least one network connection connector.

12. The installation according to claim 1, **characterized in that** the one or more fluid supply lines (14) comprise an oxygen supply line, an air supply line, a N2O or N2O/O2 mixture supply line, and/or a vacuum supply line.

13. The installation according to any one of the preceding claims, **characterized in that** the control means (4) of the box (3) are configured to process the pressure measurements from the sensors (5) and to detect an excessive or insufficient pressure in one or more of the fluid supply lines (14).

14. The installation according to claim 13, **characterized in that** the control means (4) of the box (3) are configured to trigger an alarm in case of detection of an excessive or insufficient pressure in one or more of the fluid supply lines (14).

15. The installation according to claims 1 and 14, **characterized in that** the control means (4) are configured to command a display on the box (3) and/or to transmit alarm information to said remote monitoring equipment (50).
